# EUROPEAN PATENT SPECIFICATION

(11) **EP 2 173 196 B1**
(45) Date of publication and mention of the grant of the patent: **16.02.2011**
(21) Application number: 08766875.2
(22) Date of filing: 04.07.2008
(51) Int. Cl.: A23L 1/30, A23L 1/308, A61K 31/7024, A61K 31/715, A61K 35/74

(54) **USE OF PROBIOTICS AND FIBERS FOR DIARRHOEA**
VERWENDUNG VON PROBIOTIKA UND BALLASTSTOFFEN BEI DIARRHÖ
UTILISATION DE PROBIOTIQUES ET DE FIBRES POUR LA DIARRHÉE

(30) Priority: 06.07.2007 EP 07111951
(43) Date of publication of application: 14.04.2010
(73) Proprietor: N.V. Nutricia, 2712 HM Zoetermeer (NL)
(72) Inventor: BINDELS, Jacques, NL-2441 AE Nieuwveen (NL); GOEDHART, Anna Christina, NL-3043 PR Rotterdam (NL)
(74) Representative: Ketelaars, Maarten F.J.M.
(86) International application number: PCT/NL2008/050454
(87) International publication number: WO 2009/008717

(56) References cited:
- EP-A- 0 904 784
- WO-A-2005/039319
- WO-A-2006/046871
- WO-A-2006/091103
- US-A1- 2007 218 164
- US-A1- 2007 218 170
- US-B1- 6 241 983

## Description

### FIELD OF THE INVENTION

The invention is in the field of infant nutrition comprising probiotics and fibers for prevention and/or treatment of diarrhoea.

### BACKGROUND OF THE INVENTION

Diarrhoea is a serious public health problem, a significant contributor to malnutrition, and associated with one-fourth of all deaths in children less than 5 years, especially infants, in developing countries. Each year, diarrhoea causes more than 1 billion episodes of illness, with a global average of 3 episodes per child and nearly 5 million deaths worldwide.

Current recommendations state that management of acute diarrhoea includes replacement of fluid and electrolytes loss along with nutritional support, especially in infants. But this treatment does not shorten the duration of diarrhoea. Options to shorten the duration of diarrhoea include the use of antibiotics, anti-motility and anti-secretory drugs. But this is not always effective and is often followed by serious side effects. There is general consensus that pharmacologic agents are unnecessary in treatment of acute gastroenteritis in infants.

Probiotics, especially lactobacillus species, have been used as prophylaxis as well as therapy to hasten the curing of diarrhoea.

EP 0904784 relates to a nutritional preparation for the prevention and treatment of disorders of the gastrointestinal tract, comprising a *Bifidobacterium, Enterococcus faecium* and a *Lactobacillus* strain, and preferably containing prebiotic compounds. The preparation can be in the form of a food supplement, a ready-to-use food composition, an infant formula or a tube feeding, optionally comprising fiber.

WO 2006/046871 relates to compositions comprising *L. rhamnosus* and optionally fibers for the treatment and/or prevention of sepsis, bacteraemia and/or endotoxaemia. US 6,241,983 relates to compositions for promoting gastrointestinal health containing probiotics and dietary fiber. Preferred beneficial human intestinal microorganisms include lactobacilli and bifidobacteria.

WO 2006/110406 relates to compositions comprising a probiotic component and a sweetener component.

WO 2005/039319 relates to compositions comprising mixtures of non-digestible oligosaccharides and *Bifidobacterium breve.*

WO 2006/091103 relates to compositions comprising *Bifidobacterium breve,* two non-digestible oligosaccharides and optionally *Lactobacillus paracasei.*

### SUMMARY OF THE INVENTION

The inventors have found that a composition comprising *L. rhamnosus* in combination with inulin and soy polysaccharides (soy PS) has a beneficial effect on shortening of the duration of diarrhoea at least similar to that reported for *L. rhamnosus* alone, while at the same time restoring the microbiota by offering fermentable substrates to the colon, restoring mucosal atrophy caused by the diarrhoea, and restoring nutritional deficits. The combination of both *L. rhamnosus* and inulin and soy PS will give more advantages in reducing diarrhoea in infants, improved survival of live bacteria in food products with, as a consequence, prolonged shelf life, an increased number of ingested bacteria reaching the colon in a viable form, stimulation in the colon of the growth and implantation of both exogenous and endogenous bacteria, and activation of the metabolism of these bacteria, and exerting an action along the small intestine and the colon.

The presence of the insoluble soy polysaccharides advantageously sequesters water from the liquid stools. A composition comprising a combination of *L. rhamnosus,* inulin and soy polysaccharides advantageously combines the shortening of diarrhoea effects, such as stimulation of the immune system, improved survival of the probiotic strain, improvement of the microbiota, anti-pathogenic effects, improved mineral retention, improved water retention, improved intestinal wall repair and/or restoration of nutritional deficits. The effects exceed the effects of the single components.

### DETAILED DESCRIPTION OF THE INVENTION

The present invention relates to a method for treatment and/or prevention of diarrhoea in a human subject, in particular acute diarrhoea, said method comprising administering a composition comprising *Lactobacillus rhamnosus,* inulin and soy polysaccharides to said human subject. In other words the present invention relates to the u se of a composition comprising *Lactobacillus rhamnosus,* inulin and soy polysaccharides for the manufacture of a nutritional composition for treatment and/or prevention of diarrhoea in a human subject. The present invention can also be worded as a composition comprising *Lactobacillus rhamnosus,* inulin and soy polysaccharides for treatment and/or prevention of diarrhea, in particular acute diarrhoea, in a human subject.

### Lactobacillus rhamnosus

The present composition comprises *Lactobacillus rhamnosus.* Of the lactobacilli species present in the human intestines this species is one of the most abundant and the strains belonging to this species have been found effective in prevention and/or treatment of diarrhoea, including acute diarrhoea in infants. The mechanism of prevention or treatment of diarrhoea is alleged to involve stimulation of the immune system, improvement of the microbiota and/or direct anti-pathogenic effects. Preferably the present *Lactobacillus rhamnosus* has at least 95, more preferably at least 97, even more preferably at least 99 % identity of the 16S rRNA sequence when compared to the type strain of L. rhamnosus ATCC 7469 (Stackebrandt & Goebel; 1994, Int. J. Syst. Bacteriol. 44:846-849). *L. rhamnosus* exerts its anti-diarrhoeal effect in the small intestine as well as in the colon. *L. rhamnosus* is preferred over the use of bifidobacteria, since it exerts its action also in the small intestine, the location where the infections causing diarrhoea often takes place, whereas bifidobacteria are active in the large intestine only. So, using *L. rhamnosus* instead of bifidobacteria has the advantage that the diarrhoea is also contested in the small intestine. Of the lactobacilli species *L. rhamnosus* is preferred, since strains belonging to this species are the most abundant in the intestine, the most robust and the most effective against diarrhoea.

Preferred *L. rhamnosus* strains are those isolated from the faeces of healthy human milk-fed infants. Several *L. rhamnosus* strains are commercially available from producers of lactic acid bacteria, but they can also be directly isolated from faeces, identified, characterized and produced. According to a preferred embodiment, the present composition contains at least one *L. rhamnosus* selected from the group consisting *of L. rhamnosus* LCS-742 (Morinaga), *L. rhamnosus* LR-35 (Chr. Hansen). *L. rhamnosus* GG (Valio), *L. rhamnosus* HN001 (Howaru), *L. rhamnosus* R1039, R0011, R0049 (Lallemand), *L. rhamnosus* LBA 12.5U, LR-32 (Rhodia), *L. rhamnosus* 271 231 (Probi AB), *L. rhamnosus* KC1 (DSM), *L. rhamnosus* LMG P-22799. More preferably, the *L. rhamnosus* is the strain Lactobacillus GG (ATCC 53103). More preferably the *L. rhamnosus* is the strain LMG P-22799. *L. rhamnosus* strain LMG P-22799 is a strain showing good technological properties, not unwanted antibiotic resistances, a high survival in the gastro-intestinal tract, good adherence to intestinal cells and good prevention of adhesion of intestinal pathogens to intestinal cells.

The present composition preferably comprises 10² to 10¹² colony forming units (cfu) of *L. rhamnosus* per gram dry weight of the present composition, preferably 10⁴ to 10¹¹, more preferably 10⁵ to 10¹⁰, most preferably from 10⁵ to 5x10⁹. Per 100 ml ready to use composition the present composition preferably comprises 10³ to 10¹³ cfu of the *L. rhamnosus,* preferably 10⁴ to 10¹², more preferably 10⁵ to 10¹¹, most preferably from 10⁶ to 5x10¹⁰ cfu. The dose of *L. rhamnosus* according to the present invention is preferably administered at a daily dose of 10² to 10¹³, more preferably from 10⁵ to 10¹¹, most preferably from 10⁷ to 5x10¹⁰ cfu.

### Inulin

The present composition comprises inulin. Inulin is a water soluble fiber which can be fermented by the human microbiota. Inulin is herein defined as a non-digestible oligosaccharide comprising a chain of β linked fructose units with a degree of polymerization (DP) of 2 to 250, with over 90% of the fructose units linked with a beta (2→1) linkage. The fructose chain may or may not be terminated by a glucose unit. Inulin can thus be described as GFn or Fn wherein G represents a glucosyl unit, F represents a fructosyl unit and n is the number of fructosyl units linked to each other, n being 2 or more. Preferably, the average DP of the inulin used in the present invention is 7 or more. The average DP is preferably at least 10, more preferably at least 20. A suitable inulin is for example the commercially available as Raftiline ST, RaftilineHP (Orafti) or Frutafit (Sensus). The use of inulin with an average DP above 7, more preferably 10, even more preferably 20 decreases the osmotic load in the intestine compared to inulin with a smaller average DP, thereby preventing deleterious laxative effects, which are especially unwelcome when suffering from diarrhoea.

The presence of inulin selectively stimulates the growth and/or activity of intestinal bacteria, especially of lactobacilli. This stimulation especially occurs in the colon. Thereby advantageously the pH is lowered and organic acids such as lactate and short chain fatty acids are formed (including especially butyrate) which have antipathogenic effects (including pathogens causing diarrhoea), and serve as a fuel for the enterocytes (thereby restoring the intestinal wall), and which aid in mineral retention which is especially advantageous during diarrhoea. Additionally inulin stimulates the growth, survival and/or metabolic activity of the probiotic strain *L. rhamnosus* along the intestinal tract. This includes not only the large intestine, but also advantageously the small intestine.

The present nutritional composition preferably comprises at least 0.5 mg of inulin per g dry weight of the composition, preferably at least 1.5 mg, more preferably at least 5 mg, even more preferably at least 10 mg. Preferably, the present composition does not comprise more than 0.5 g inulin per g dry weight of the composition, more preferably not more than 0.35 g, even more preferably not more than 0.2 g, most preferably not more than 0.1 g. The present nutritional composition preferably comprises at least 5 mg of inulin per 100 ml of the present liquid composition, preferably at least 15 mg, more preferably at least 50 mg per 100 ml, even more preferably at least 100 mg. Preferably, the present composition does not comprise more than 10 g inulin per 100 ml, more preferably not more than 5 g per 100 ml, even more preferably not more than 2 g per 100 ml, most preferably not more than 1 g per 100 ml. The present inulin is preferably administered in a daily dose of 0.1 to 10 g, more preferably 0.2 to 5 g, more preferably 0.5 to 3 g.

### Soy polysaccharides

The present composition comprises soy polysaccharides. In the present invention the term soy polysaccharide (PS) and soy fibre can be used interchangeably and are defined as the non-starch, largely insoluble polysaccharides isolated from soy bean. When referring to soy polysaccharides in the present invention, polysaccharides are defined as saccharides with a DP above 10, more preferably above 20, more preferably above 50, even more preferably above 100. A suitable source for soy fibre is Fibrim (Solae). Soy polysaccharides contain both cellulosic and non-cellulosic dietary fiber. Typically commercially available sources of soy polysaccharides suitable for the present invention comprise, based on the official AOAC method, 72-78% total dietary fiber and the fiber component consists of about 94-95 % water insoluble fiber (cellulose and hemicellulose) and about 5-6 % water soluble fiber (arabinogalactan like polysaccharides).
The presence of the insoluble soy polysaccharides advantageously sequesters water from the liquid stools. Furthermore, the presence of soy PS advantageously results in the formation of butyrate. Butyrate is a fuel for enterocytes and helps in repairing intestinal damage caused by the inflammatory reactions against the pathogens causing diarrhoea.

The present nutritional composition preferably comprises at least 0.8 mg of soy PS per g dry weight of the composition, preferably at least 2.5 mg, more preferably at least 8 mg, even more preferably at least 15 mg per g dry weight. Preferably, the present composition does not contain more than 0.8 g soy PS per g dry weight of the composition, more preferably not more than 0.6 g, even more preferably not more than 0.35 g, most preferably not more than 0.15 g per g dry weight. The present nutritional composition preferably comprises at least 8 mg of soy PS per 100 ml of the present liquid composition, preferably at least 25 mg, more preferably at least 80 mg per 100 ml, even more preferably at least 150 mg per 100 ml. Preferably, the present composition does not contain more than 15 g soy PS 100 ml, more preferably not more than 8 g per 100 ml, even more preferably not more than 3 g per 100 ml, most preferably not more than 1.5 g per 100 ml. The present soy PS is preferably administered in a daily dose of 0.15 to 15 g, more preferably 0.3 to 8 g, more preferably 0.8 to 5 g.

Preferably the weight ratio soy PS : inulin is between 5 : 0.2, more preferably between 3 : 0.5, even more preferably between 2 : 1. A balanced ratio between inulin and soy PS is advantageous since it ensures a proper ratio of soluble and insoluble fibers and their different beneficial effects on diarrhoea. For the sake of clarity it is noted that "weight ratio soy PS : inulin is between 5 : 0.2" means that the weight of soy PS divided by the weight of inulin gives a number that lies between 5 and 0.2.
A composition comprising a combination of *L. rhamnosus,* inulin and soy polysaccharides advantageously combines the shortening of diarrhoea effects, such as stimulation of the immune system, improved survival of the probiotic strain, improvement of the microbiota, anti-pathogenic effects, improved mineral retention, improved water retention, improved intestinal wall repair and/or restoration of nutritional deficits, along the entire intestinal tract. The effects are assumed to exceed the effects of the single components. Especially the combination of *L. rhamnosus,* soy PS and inulin results in an increased formation of short chain fatty acids, including butyrate, and lactate, and an improved action in the small intestine. Preferably the composition comprises 10⁴ to 10¹² colony forming units (cfu) *of L. rhamnosus* per gram soy PS plus inulin, preferably 10⁶ to 10¹¹, more preferably 10⁷ to 10¹⁰, most preferably from 10⁸ to 5x10⁹ cfu.

### Micronutrients

The present composition preferably comprises zinc. Zinc deficiency is highly prevalent in children in developing countries. Zinc supplements given during diarrhoea reduce the duration and severity of treated episodes. During diarrhoea generally an overproduction of nitric oxide (NO) occurs, which induces secretion and cellular damage as a free radical. Addition of zinc may contribute to improving the physiologic status of the small intestine and potentially reduce the risks of recurrent diarrhoea episodes. Preferably the composition comprises at least 35 µg zinc per g dry weight of the composition, more preferably at least 60 µg. Preferably the composition does not comprise more than 250 µg zinc per g dry weight of the composition.
The present composition preferably comprises iron to replace iron loss. Furthermore, iron reduces the duration of diarrhoea. Preferably the composition comprises at least 40 µg per g dry weight, more preferably at least 80 µg. Preferably the composition does not comprise more than 250 µg iron per g dry weight of the composition.
Preferably the composition comprises both zinc and iron, since a simultaneous supplementation of zinc and iron was found to reduce the risk of severe diarrhoea. Preferably the weight ratio of iron to zinc is between 0.8 and 1.3. A balanced ratio of zinc and iron ensures a proper absorption of both zinc and iron.

### Nutritional composition

The present composition is particularly suitable for providing the daily nutritional requirements to an infant with the age below 36 months, particularly an infant with the age below 24 months, even more preferably an infant with the age below 12 months. Hence, the present composition preferably comprises a lipid, protein and digestible carbohydrate component wherein the lipid component provides 20 to 55% of the total calories, the protein component provides 5 to 15% of the total calories and the digestible carbohydrate component provides 30 to 75% of the total calories. Preferably the present composition comprises a lipid component providing 25 to 50 % of the total calories, a protein component providing 6 to 13% of the total calories and a digestible carbohydrate component providing 40 to 65% of the total calories.

When in liquid form, e.g. as a ready-to-feed liquid, the composition preferably comprises 1.0 to 6.5 g fat per 100 ml, more preferably 1.8 to 4.0 g per 100 ml. Based on dry weight the present composition preferably comprises 8 to 40 wt.% fat, more preferably 12 to 30 wt.%. The amount of saturated fatty acids is preferably below 58 wt.% based on total fatty acids, more preferably below 45 wt.%. The concentration of monounsaturated fatty acids preferably ranges from 17 to 60% based on weight of total fatty acids. The concentration of polyunsaturated fatty acids preferably ranges from 20 to 60% based on weight of total fatty acids. Preferably the composition comprises the n-6 polyunsaturated fatty acid linoleic acid (LA) and the n-3 polyunsaturated fatty acid α-linolenic acid (ALA). Preferably the weight ratio LA/ALA is between 4 and 10 more preferable between 5 and 7.
Preferably the composition comprises long chain poly-unsaturated fatty acids, such as arachidonic acid, docosahexaenoic acid and/or eicosapentaenoic acid. Preferably n-3 LC-PUFA are present, such as EPA and/or DHA. n3-LC-PUFA reduce intestinal inflammatory responses, which is especially advantageous in infants suffering from diarrhoea.

When in liquid forum, e.g. as a ready-to-feed liquid, the composition preferably comprises 0.5 to 4 g protein per 100 ml, more preferably 1.0 to 3.0 g per 100 ml. Based on dry weight the present composition preferably comprises 5 to 30 wt.% protein, more preferably 10 to 20 wt.%. The present composition preferably comprises at least 50 wt.% protein derived from non-human milk based on total protein, more preferably at least 90 wt.%. Preferably the present composition comprises at least 50 wt.% cow milk derived protein based on total protein, more preferably at least 90 wt.%. The present composition preferably comprises casein and/or whey proteins. Preferably the weight ratio casein:whey protein is 0: 100 to 100:0, more preferably 10:90 to 90:10, more preferably 20:80 to 80:20. The term protein as used in the present invention refers to the sum of proteins, peptides and free amino acids. The composition may optionally comprise hydrolysed proteins and/or free amino acids. Preferably the composition does not comprise hydrolysed proteins and/or free amino acids, since this increases the osmotic load of the composition which is undesirable for human subjects suffering from diarrhoea.

When in liquid form, e.g. as a ready-to-feed liquid, the composition preferably comprises 2 to 30 g digestible carbohydrates per 100 ml, more preferably 5 to 15 g per 100 ml. Based on dry weight the present composition preferably comprises 40 to 80 wt. % digestible carbohydrate, more preferably 50 to 70 wt.%. Preferably the composition comprises at least one digestible carbohydrate selected from the group consisting of lactose, maltodextrin, starch, saccharose, glucose, and maltose. Preferably the present composition comprises maltodextrin and/or starch. Using digestible carbohydrates with a higher degree of polymerization instead of mono- and disaccharides reduces the osmotic load, which is advantageous in human subjects suffering from diarrhoea.
The present composition preferably comprises rice starch. Rice-based oral rehydration solutions (ORS) have found to be more effective in decreasing stool output, reducing duration of diarrhoea, and improving intestinal absorption of fluid and electrolytes in children with acute infectious diarrhoea compared with glucose-based ORS. The benefits of rice-based ORS have previously been attributed to its lower osmotic load, faster absorption, and higher carbohydrate content. Another explanation for the reduction of stool output and the improvement in absorption with rice-based ORS may be the ability of rice to inhibit intestinal excretion.
The present composition preferably comprises pectin or pectin degradation products. Pectin and/or its degradation products advantageously inhibit the adhesion of pathogenic micro-organisms causing diarrhoea to the intestinal wall, thereby reducing diarrhoea.
Preferably the composition comprises galacto-oligosaccharides (GOS), more preferably β-linked GOS. These β-linked GOS beneficially reduce the adherence of diarrhea causing bacteria to intestinal cells. Preferably the composition comprises 10 to 200 mg β-linked GOS per g dry weight, more preferably 20 to 90 mg. A suitable source of β-linked GOS is Vivinal (Borculo).
Preferably the composition comprises nucleotides. Dietary nucleotides advantageously improve the repair of the intestinal cells, damaged by the pathogens causing diarrhoea.

The present composition is preferably administered in liquid form. In order to meet the caloric requirements of the infant, the composition preferably comprises 50 to 200 kcal/100 ml liquid, more preferably 60 to 90 kcal/100 ml liquid, even more preferably 60 to 75 kcal/100 ml liquid. This caloric density ensures an optimal ratio between water and calorie consumption. The osmolarity of the present composition is preferably between 150 and 420 mOsmol/l, more preferably 260 to 320 mOsmol/l. This osmolarity is of utmost importance for infants suffering from diarrhoea and aids to reduce the gastrointestinal stress and to decrease diarrhoea.

Preferably the composition is in a liquid form, with a viscosity below 35 cps as measured in a Brookfield viscometer at 20°C at a shear rate of 100 s⁻¹. Suitably, the composition is in a powdered from, which can be reconstituted with water to form a liquid, or in a liquid concentrate form, which should be diluted with water. When the composition is a liquid form, the preferred volume administered on a daily basis is in the range of about 80 to 2500 ml, more preferably about 450 to 1000 ml per day. Suitably the composition is reconstituted with water to form a semi-liquid, spoonable composition.

In a preferred embodiment, the composition is a rehydration solution comprising *L*. *rhamnosus,* inulin, soy PS, glucose and sodium, potassium, chloride with an osmolarity between 200 and 320 mOsmol/l, more preferably between 230 and 285 mOsmol/l, most preferably between 235 and 245 mOsmol/l,

### Application

The composition according to the present invention has been found to be particularly useful as an infant nutrition, in particular for prematurely born babies, term born babies, as well as infants which are in the weaning period to solid food. Hence the present invention provides a method for providing nutrition to a human infant, said method comprising administering to the infant the present composition. Preferably the infant has an age between 0 and 36 months, even more preferably between 0 and 18 months, most preferably between 0 and 12 months. The present composition can be advantageously used in the manufacture of a medicament for use in the prevention and/or treatment of diarrhoea, in particular infectious diarrhoea, more particular rotavirus diarrhoea. Preferably the composition comprising *L. rhamnosus,* inulin and soy PS is used to treat acute diarrhoea.

In a preferred embodiment the combination of *L. rhamnosus,* inulin and soy PS is present in an oral rehydration solution further comprising glucose and electrolytes in a first rehydration step to restore water and electrolyte balance, while in the mean time immediately establishing the presence of *L. rhamnosus,* inulin and soy PS in the gastro-intestinal tract.

The present composition is preferably used after a first rehydration step of about 3 to 4 h with a glucose-electrolyte mixture to restore the water and electrolyte balance. The combination *of L. rhamnosus,* inulin, soy PS, together with a lipid component, a digestible carbohydrate component and a protein component quickly restores normal stool output and consistency, while at the same time providing nutrition for growth and restoration of especially the small intestinal wall. Preferably the present composition is used for 2-10, more preferably 2-5 days until the diarrhoea has resolved.

In this document and in its claims, the verb "to comprise" and its conjugations is used in its non-limiting sense to mean that items following the word are included, but items not specifically mentioned are not excluded. In addition, reference to an element by the indefinite article "a" or "an" does not exclude the possibility that more than one of the element is present, unless the context clearly requires that there be one and only one of the elements. The indefinite article "a" or "an" thus usually means "at least one".

### EXAMPLES

### Example 1: Nutritional supplementation with a mixture of L. rhamnosu,s inulin and soy polysaccharides and micronutrients to reduce the duration of acute infantile diarrhoea

### Methods:

A randomized, double blind clinical trial was conducted to assess the efficacy of an infant formula containing *Lactobacillus rhamnosus* (probiotic) and dietary fiber (inulin and soy polysaccharides) for management of 58 Indonesian well-nourished male infants aged 3-12 months suffering from acute diarrhoea with moderate dehydration. After adequate oral rehydration, the patients were randomly assigned to receive either a low lactose infant formula containing a combination of *Lactobacillus rhamnosus* (5*10⁸ per 100 ml), dietary fiber (0.15 g inulin/100 ml and 0.25 g soy polysaccharides/100 ml) and enhanced concentration of a number of particular micronutrients (1.1 mg iron/100 ml, 0.9 mg zinc/100ml, 0.05 mg copper/100 ml, 7 µg manganese/100 ml, 1.0 µg selenium/100 ml, 1.9 mg vitamin D3/100ml and 1.1 µg vitamin E/100 ml) (study group) or a low lactose infant formula without *Lactobacillus rhamnosus* or dietary fiber (control group) and with standard micronutrient concentrations (0.5 mg iron/100 ml, 0.5 mg zinc/100ml, 0.04 mg copper/100 ml, 1.4 mg vitamin D3/100ml and 0.8 µg vitamin E/100 ml) (control group). The following data were recorded daily for each infant: stool volume, stool frequency and consistency, and accurate body weight measurement. The duration of diarrhoea was defined as the number of hours after admission until excretion of the last liquid or semiliquid stool that is not followed by another abnormal stool within 24 hours.

### Results:

The patients in the two groups were comparable on admission with respect to age, anthropometric indicators and nutritional status, clinical features, medication history and laboratory values that could be associated with diarrhoeal morbidity. The mean (SD) age was 8.1 (2.6) months in the study group and 8.0 (2.7) months in the control group. On admission, the patients had moderate dehydration status. No differences were detected between study and control groups in the isolation of pathogens. Rotavirus was the most prevalent etiological factor in 76% of the cases, and it was found equally in the study and control groups.
The duration of diarrhoea was significantly shorter in the study group than in the control group (1.63 versus 2.45 days; p<0.05; for the study and control group respectively), and this became obvious after the first day of treatment. The present study supports the evidence that an anti-diarrhoeal formula containing probiotics, prebiotics and micronutrients after oral rehydration has a beneficial effect in shortening the duration of infantile diarrhoea in developing countries. There was a trend towards lower stool weight in the study group compared to the control group. The study group tend ed to have lower stool output (in grams per kilogram body weight per day) after dietary treatment compared to the control group, already during the first and second day after rehydration in the hospital.

### Example 2: Synergistic effect of soy PS, inulin and Lactobacillus rhamnosus.

### Materials:

An *in vitro* semi-dynamic batch fermentation system was used, using toddler faeces.
As a source of Soy PS Fibrim (The Solae Company) was used. As a source of inulin RaftilinST (Orafti) was used, As a source of *L. rhamnosus* L GG (Valio) was used.
Fresh faeces was obtained from healthy toddlers with an age of 2 years.
Experimental medium was McBain & MacFarlane medium (Buffered peptone water 3.0 g/l, yeast extract 2.5 g/l, Tryptone 3.0 g/l, L-Cysteine-HCl 0.4 g/l, bile salts 0.05 g/l, K₂HPO₄.3H₂O 2.6 g/l, NaHCO₃ 0.2 g/l, NaCl 4.5 g/l, MgSO₄.7H₂O 0.5 g/l, CaCl₂ 0.228 g/l, FeSO₄ 0.005 g/l)

### Methods:

Fresh faecal material was mixed with McBain & MacFarlane medium, which is representative for the intestinal environment, in a weight ratio of 1:5.
At t = 0, 6 ml of the faecal suspension was mixed with fibres and/or L GG and transferred into a dialysis tube in a 100 ml bottle filled with buffered dialysis medium (K.HPO₄.3H₂O 2.6 g/l, NaHCO₃ 0.2 g/l, NaCl 4.5 g/l, MgSO₄.7H₂O 0.5 g/l, CaCl₂ 0.228 g/l, FeSO₄.7H2O 0.005 g/l, pH 6.3). The bottle was closed and incubated at 37 °C. The mixtures of fibres and L GG were as follows:

| Mixture: | 1 | 2 | 3 | 4 | 5 | 6 |
|---|---|---|---|---|---|---|
| Soy PS | 0 | 192mg | 0 | 0 | 120mg | 120mg |
| Inulin | 0 | 0 | 192mg | 0 | 72mg | 72mg |
| L GG | 0 | 0 | 0 | 1.2x10⁹cfu | 0 | 1.2x10⁹cfu |

Samples of 0.5 ml were taken from the dialysis tube and from the dialysis buffer with a hypodermic syringe after 48 h and stored at -18°C. Experiments were performed in duplicate and all handlings were performed in an anaerobic cabinet.
Lactate was determined enzymatically, using a lactic acid detection kit with D- and L-lactate-dehydrogenase (Raisio Diagnostics Spa, Rome, Italy). Short chain fatty acids were detected with gas chromatography: SCFA were extracted in MilliQ. 2-ethyl-butyrate was used as an internal standard. Samples were analyzed on a capillary column (Restek Stabilwax DA 15m x 0.53 x 1.0 µm; ZB-FFAB 15mx0.13x1.0µm) with FID detector. The mobile phase was helium.

### Results and Conclusions:

The results are expressed as amounts of mmol lactate or SCFA formed per g of fibre and shown in Table 1.

**Table 1 Organic acids (in mmol/g fibre) formed upon fermentation of different fibre mixtures.**

| Mixture | 1 | 2 | 3 | 4 | 5 | 6 |
|---|---|---|---|---|---|---|
| Lactate | 0.45 | 1.19 | 6.22 | 0.19 | 1.30 | 1.88 |
| SCFA | 7.11 | 19.09 | 25.42 | 7.01 | 21.80 | 23.53 |
| Acetate | 3.18 | 10.65 | 17.59 | 3.21 | 11.81 | 13.56 |
| Propionate | 1.22 | 2.62 | 0.62 | 1.10 | 2.89 | 2.84 |
| Butyrate | 0.26 | 0.21 | 0.01 | 0.21 | 0.84 | 0.30 |

These results demonstrate that *L. rhamnosus* alone does not improve the intestinal fermentation. A combination of soy PS with inulin shows a higher lactate and SCFA formation than when using soy PS alone. The presence of *L rhamnosus* further enhances the lactate and SCFA formation of the soy PS and inulin mixture. When using inulin alone, a high lactate and SCFA formation is observed. However, almost no butyrate is formed. Having inulin present in a mixture together with soy PS or soy PS and *L. rhamnosus* advantageously improves the formation of butyrate. Therefore a mixture of soy PS, inulin and *L. rhamnosus* shows the best fermentation characteristics for children suffering from diarrhoea.

### Example 3: Infant nutrition

Infant nutrition comprising:
- a vegetable fat component providing 27% of the total calories, with 40% polyunsaturated fatty acids based on total fatty acids and a wt. ratio LA/ALA of 5.5.
- a cow's milk protein component providing 12% of the total calories consisting of 20 wt.% whey protein and 80 wt.% casein, and
- a digestible carbohydrate component providing 61% of the total calories, comprising over 80 wt.% of maltodextrin and starch.
- *L. rhamnosus:* 5 * 10⁸ cfu per 100 ml.
- Inulin: 0.15 g per 100 ml
- Soy polysaccharides 0.25 per 100 ml

The label of the package of this infant nutrition indicates that the nutrition is to be used in case of diarrhoea.

### Example 4:

Oral rehydration solution comprising per 100 ml:
2.0 g glucose, 0.2 g citrate, 138 mg sodium, 78 mg potassium, 177 mg chloride, 1*10⁸ cfu *L. rhamnosus,* 0.05 g inulin, 0.10 g soy PS.

## Claims

1. Use of a composition comprising *Lactobacillus rhamnosus,* inulin and soy polysaccharides for the manufacture of a nutritional composition for treatment and/or prevention of diarrhoea in a human subject.

2. Use according to claim 1 wherein the weight ratio soy PS : inulin is between 5 to 0.2.

3. Use according to any of the preceding claims wherein the composition further comprises a fat component providing 20 to 55% of the total calories, a protein component providing 5 to 15% of the total calories and a digestible carbohydrate component providing 30 to 75% of the total calories.

4. Use according to any of the preceding claims wherein the human subject has an age below 36 months.

5. Use according to any of the preceding claims wherein the composition comprises 10⁴ to 10¹¹ cfu *L. rhamnosus* per g dry weight of the composition.

6. Use according to any of the preceding claims wherein the *L. rhamnosus* is *L. rhamnosus* strain LMG P-22799.

7. Use according to any of the preceding claims wherein the composition comprises 1.5 to 350 mg inulin per g dry weight of the composition.

8. Use according to any of the preceding claims wherein the composition comprises 2.5 to 600 mg soy polysaccharides per g dry weight of the composition.

9. Use according to any of the preceding claims wherein the composition further comprises at least 35 µg zinc and/or 40 µg iron per g dry weight of the composition.

10. Use according to any of claims 3-9 wherein the digestible carbohydrate component comprises maltodextrin and/or starch and wherein the protein component comprises non-hydrolysed proteins.

11. Use according to any of the preceding claims for the treatment of acute diarrhoea.

12. Use according to any of the preceding claims for the treatment of rotavirus diarrhoea.

## Patentansprüche

1. Verwendung von einer Zusammensetzung, umfassend *Lactobacillus rhamnosus,* Inulin und Soja-Polysaccharide, für die Herstellung von einer Emährungs-Zusammensetzung zur Behandlung und/oder Vorbeugung von Diarrhö bei einem menschlichen Patienten.

2. Verwendung nach Anspruch 1, wobei der Gewichts-Index Soja PS : Inulin zwischen 5 bis 0,2 liegt.

3. Verwendung nach einem der vorangehenden Ansprüche, wobei die Zusammensetzung weiterhin eine Fett-Komponente unter Bereitstellung von 20 bis 55 % von den gesamten Kalorien, eine Protein-Komponente unter Bereitstellung von 5 bis 15 % von den gesamten Kalorien und eine verdaubare Kohlenhydrat-Komponente unter Bereitstellung von 30 bis 75 % von den gesamten Kalorien umfasst.

4. Verwendung nach einem der vorangehenden Ansprüche, wobei der menschliche Patient ein Alter unter 36 Monaten aufweist.

5. Verwendung nach einem der vorangehenden Ansprüche, wobei die Zusammensetzung 10⁴ bis 10¹¹ cfu *L. rhamnosus* pro g Trockenmasse von der Zusammensetzung umfasst.

6. Verwendung nach einem der vorangehenden Ansprüche, wobei das *L. rhamnosus* L. rhamnosus-Stamm LMG P-22799 ist.

7. Verwendung nach einem der vorangehenden Ansprüche, wobei die Zusammensetzung 1,5 bis 350 mg Inulin pro g Trockenmasse von der Zusammensetzung umfasst.

8. Verwendung nach einem der vorangehenden Ansprüche, wobei die Zusammensetzung 2,5 bis 600 mg Soja-Polysaccharide pro g Trockenmasse von der Zusammensetzung umfasst.

9. Verwendung nach einem der vorangehenden Ansprüche, wobei die Zusammensetzung weiterhin mindestens 35 µg Zink und/oder 40 µg Eisen pro g Trockenmasse von der Zusammensetzung umfasst.

10. Verwendung nach einem der Ansprüche 3-9, wobei die verdaubare Kohlenhydrat-Komponente Maltodextrin und/oder Stärke umfasst und, wobei die Protein-Komponente nicht-hydrolysierte Proteine umfasst.

11. Verwendung nach einer der vorangehenden Ansprüche für die Behandlung von akuter Diarrhö.

12. Verwendung nach einem der vorangehenden Ansprüche für die Behandlung von Rotavirus-Diarrhö.

## Revendications

1. Utilisation d'une composition comprenant du *Lactobacillus rhamnosus,* de l'inuline et des polysaccharides de soja dans la fabrication d'une composition nutritionnelle pour le traitement et/ou la prévention de la diarrhée chez un sujet humain.

2. Utilisation selon la revendication 1, dans laquelle le rapport en poids entre PS de soja : inuline est compris entre 5 et 0,2.

3. Utilisation selon l'une quelconque des revendications précédentes, dans laquelle la composition comprend en outre un composant de graisse fournissant 20 à 55 % des calories totales, un composant de protéine fournissant 5 à 15 % des calories totales et un composant de carbohydrate digestible fournissant 30 à 75 % des calories totales.

4. Utilisation selon l'une quelconque des revendications précédentes, dans laquelle le sujet humain a un âge inférieur à 36 mois.

5. Utilisation selon l'une quelconque des revendications précédentes, dans laquelle la composition comprend 10⁴ à 10¹¹ cfu de *L.rhamnosus* par g de poids sec de la composition.

6. Utilisation selon l'une quelconque des revendications précédentes, dans laquelle le *L.rhamnosus* est une souche de *L.rhamnosus* LMG P-22799.

7. Utilisation selon l'une quelconque des revendications précédentes, dans laquelle la composition comprend 1,5 à 350 mg d'inuline par g de poids sec de la composition.

8. Utilisation selon l'une quelconque des revendications précédentes, dans laquelle la composition comprend 2,5 à 600 mg de polysaccharides de soja par g de poids sec de la composition.

9. Utilisation selon l'une quelconque des revendications précédentes, dans laquelle la composition comprend en outre au moins 35 µg de zinc et/ou 40 µg de fer par g de poids sec de la composition.

10. Utilisation selon l'une quelconque des revendications 3-9, dans laquelle le composant carbohydrate digestible comprend de la maltodextrine et/ou de l'amidon et dans laquelle le composant de protéine comprend des protéines non-hydrolysées.

11. Utilisation selon l'une quelconque des revendications précédentes pour le traitement de la diarrhée aigue.

12. Utilisation selon l'une quelconque des revendications précédentes pour le traitement de la diarrhée à rotavirus.
